# EUROPÄISCHE PATENTSCHRIFT

(11) **EP 2 343 022 B1**
(45) Veröffentlichungstag und Bekanntmachung des Hinweises auf die Patenterteilung: **29.05.2013**
(21) Anmeldenummer: 11150325.6
(22) Anmeldetag: 06.01.2011
(51) Int. Cl.: A61B 18/14, A61B 18/02

(54) **Ablationskatheteranordnung zur Therapie einer Herzrhythmusstörung**
Ablation catheter arrangement for treatment of a cardiac arrhythmia
Agencement de cathéter d'ablation pour le traitement d'une arythmie cardiaque

(30) Priorität: 12.01.2010 US 294116 P
(43) Veröffentlichungstag der Anmeldung: 13.07.2011
(73) Patentinhaber: VascoMed GmbH, 79589 Binzen (DE)
(72) Erfinder: Geistert, Wolfgang, 79618, Rheinfelden (DE)
(74) Vertreter: Lindner-Vogt, Karin L.

(56) Entgegenhaltungen:
- WO-A1-2009/158595
- WO-A2-2004/064657
- US-A1- 2008 161 794
- US-B1- 6 514 249

## Beschreibung

Die Erfindung betrifft eine Ablationskatheteranordnung zur Ablation von Herzgewebe zur Therapie einer Herzrhythmusstörung, insbesondere von Vorhofflattern oder -flimmern.

Es ist seit längerem bekannt und bewährt, kardiale Arrhythmien durch gezielte Verödung von Herzgewebe mit darin enthaltenen Leitungsbahnen, die sogenannte Ablation, zu therapieren. Dabei unterscheidet man nach heutigem Kenntnisstand zwischen der HF- oder RF-Ablation, der Kryoablation, der Laserablation, der Mikrowellenablation und der Ablation mit fokussiertem Ultraschall.

WO 2004/064657 beschreibt eine chururgische Vorrichtung zur RF-Ablation mit EKG- und Drücküberwachung.

Die in der klinischen Routine eingesetzten HF-Generatoren erzeugen einen hochfrequenten Wechselstrom um 500 kHz. Über den Kontakt des Ablationskatheters mit dem Gewebe, an dem dieses hochfrequente Signal für die Dauer der Applikation anliegt, kommt es zu einem lokalen Erhitzungseffekt. Dabei entstehen Temperaturen zwischen 45 und 100°C. Die Folge ist eine Gewebeläsion, die einen maximalen Durchmesser um 5 mm und eine Tiefe von bis zu 2-3 mm aufweist. Ziel der Läsion ist die Elimination des zuvor identifizierten arrhythmogenen Substrats, welches für den Tachykardie-Mechanismus verantwortlich ist. Bei der Kryoablation wird das für die Arrhythmie verantwortliche Herzmuskelgewebe gezielt unterkühlt. Kälte wird schon seit Jahrzehnten für die Behandlung von Herzrhythmusstörungen eingesetzt. Weil die Kryotherapie die Zellen gefriert - im Unterschied zu der soeben genannten Hitze basierten Radiofrequenzablation - stellt sie eine alternative Behandlungsoption für Elektrophysiologen und Herzchirurgen dar.

Die Spitze des Kryoablationskatheters wird auf Temperaturen von unter 0°C heruntergekühlt. Über die Spitze des Katheters wird dem umliegenden Gewebe Wärme entzogen. In Abhängigkeit vom verwendeten Katheter entstehen an der Katheterspitze Temperaturen von -75°C oder sogar kälter. Vom Patienten wird diese Kälte nicht wahrgenommen.

Die für die Leitung der Arrhythmie verantwortlichen Herzmuskelzellen werden durch die Kälteeinwirkung so verändert, dass sie elektrische Erregungen nicht mehr leiten können.

Während einer kardiologischen Ablationsprozedur muss regelmäßig, besser ständig, geprüft werden, ob die zu behandelnde Herzrhythmusstörung beseitigt ist. Traditionell wird die Erfolgskontrolle mittels EKG-Messung und/oder intrakardialer Blutdruckmessung durchgeführt. Die Blutdruckmessung ist insbesondere bei Ablation von tachykarden Vorhofrhythmusstörungen (Vorhofflattern, Vorhofflimmern) von Nutzen, da durch Blutdruckmessung im Vorhof einfach festgestellt werden kann, ob die Pumpfunktion der Vorhöfe wiederhergestellt ist. Die Blutdruckmesseinrichtung ist eine zur traditionellen Ablationsanordnung (Ablationsgerät und optionales EKG-Messgerät) zusätzliche Einrichtung, die einen zusätzlichen Blutdruckmesskatheter erfordert, welcher in den Vorhöfen platziert wird. Dieser Blutdruckmesskatheter ist notwendig, da die kleinen Vorhofdrücke mit einer traditionellen externen Blutdruckmessung nicht gemessen werden können.

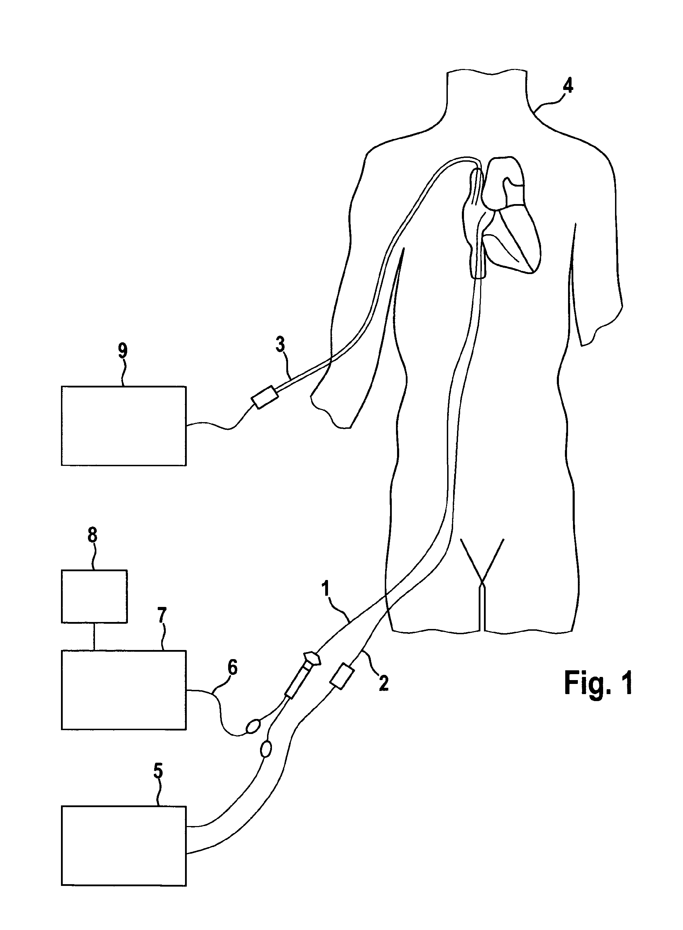

Fig. 1 zeigt eine Geräte- und Katheteranordnung zur HF-Katheterablation der traditionellen Art. Neben dem Ablationskatheter 1 sind mindestens ein weiterer EP-Diagnosekatheter 2 und ein Blutdruckmesskatheter 3 im Herz des Patienten 4 platziert. Der Ablationskatheter 1 und der EP-Diagnosekatheter 2 sind an ein EKG-Messsystem 5 angeschlossen. Der Ablationskatheter 1 verfügt über Spülöffnungen, welche dafür gedacht sind, Flüssigkeit an der Ablationsstelle abgeben zu können, um das Gewebe an dieser Stelle zu kühlen. Die Spülöffnungen sind mit einem Spülflüssigkeitsschlauch 6 verbunden, der an seinem proximalen Ende an eine Pumpe 7 angeschlossen ist, welche für die Flüssigkeitszufuhr aus einem Reservoir 8 sorgt.

Der Blutdruckmesskatheter 3 ist an ein Blutdruckmessgerät 9 angeschlossen. Über die im Blutdruckmesskatheter 3 befindliche Flüssigkeitssäule werden Blutdruckänderungen nach außen geführt und mit dem Blutdruckmessgerät 9 gemessen.

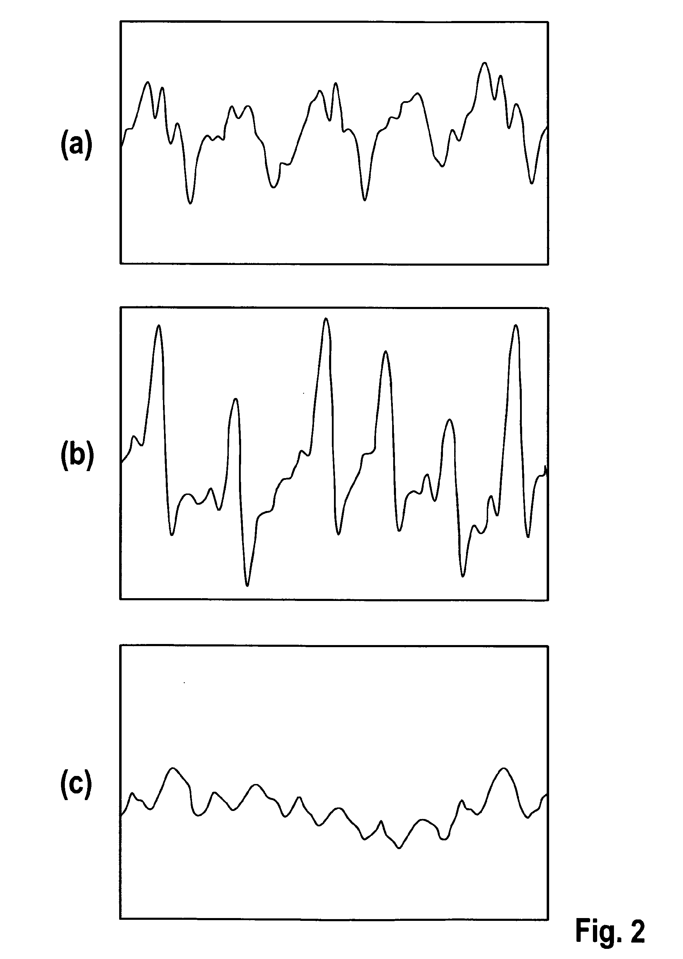

Fig. 2 zeigt eine Vorhof-Blutdruckkurve während Sinusrhythmus (a) und Kurven während Vorhof-Flimmem bzw. -Flattern (b)+(c). Es ist deutlich zu sehen, dass der Unterschied eine einfache Ablationserfolgskontrolle ermöglicht.

Es ist eine Aufgabe der Erfindung, eine vereinfachte Ablationskatheteranordnung zur Ausführung der genannten Therapie bereitzustellen, welche insbesondere eine einfachere und schnellere Einführung in den Körper eines Patienten und Handhabung während eines Eingriffs ermöglicht.

Diese Aufgabe wird durch eine Anordnung mit den Merkmalen des Anspruchs 1 gelöst.

Gemäß einem Aspekt der Erfindung umfasst die vorgeschlagene Anordnung eine mit dem Ablationsgenerator und der Spülflüssigkeitsquelle verbindbaren Ablationskatheter mit einem Ablationsbereich, mindestens einer Spülöffnung sowie einem Spülkanal zum Zuführen von Spülflüssigkeit in den Behandlungsbereich. Des Weiteren umfasst sie ein mit dem Spülkanal des Ablationskatheters in Fluidverbindung stehendes und zur Erfassung des intrakardialen Blutdrucks über die Flüssigkeitssäule der Spülflüssigkeit ausgebildetes Blutdruckmessgerät. Die direkte Anbindung des Blutdruckmessgerätes an den Ablationskatheter ermöglicht eine Vereinfachung der Anordnung dadurch, dass ein gesonderter Blutdruckmesskatheter und der mit dessen Bereitstellung, Einführung, Handhabung, Herausführung aus dem Körper und Entsorgung verbundene Aufwand verzichtbar werden.

Der Spülkanal des Ablationskatheters kann ein aus bestimmten Gründen bereits vorhandener Flüssigkeitskanal sein. Bei HF-Ablationskathetern kann dieser ein Flüssigkeitskanal sein, durch den Kühlflüssigkeit zum Behandlungsbereich geleitet wird. Bei allen Ablationskathetem kann dieser ein Flüssigkeitskanal sein, durch den ein Führungsdraht geschoben werden kann, der zur Steuerung des Ablationskatheters und/oder zur Hinführung des Ablationsbereichs des Ablationskatheters zum Behandlungsbereich dient.

Bei einer traditionellen Geräte- und Katheteranordnung kann zusätzlich weiterhin ein EKG-Messgerät vorgesehen sein, auf das bei einem rein anatomischen Behandlungsansatz auch verzichtet werden kann.

Die Erfindung sieht vor, dass die Spülflüssigkeitsquelle eine Spülflüssigkeitspumpe aufweist und eine Förder-/Mess-Steuereinheit zur Steuerung einer zeitlich abgestimmten Förderung von Spülflüssigkeit und Messung des Blutdrucks vorgesehen ist. Die Steuereinheit kann in einer ersten Ausgestaltung in die Spülflüssigkeitspumpe integriert sein. In weiteren Ausführungen ist sie in das Blutdruckmessgerät integriert, oder sie ist als separate Einheit ausgeführt und mit der Spülflüssigkeitspumpe und dem Blutdruckmessgerät über Mittel zur Datenkommunikation, insbesondere Signalleitungen, verbindbar. Die erwähnten baulichen Ausgestaltungen können in einem konkreten Geräteset jeweils bestimmte Vorteile aufweisen, wie etwa Vereinfachungen der Handhabung bei baulicher Integration mehrerer Komponenten oder aber eine höhere Flexibilität, bei Verzicht auf eine solche bauliche Integration.

Eine weitere Ausführung der Erfindung ist mit einem verzweigten Spülflüssigkeitsschlauch zur dauerhaften Fluidverbindung des Ablationskatheters sowohl mit dem Blutdruckmessgerät als auch der Spülflüssigkeitsquelle, insbesondere der Spülflüssigkeitspumpe, versehen. Der Schlauch verbindet also hier die Spülflüssigkeitsquelle und das Blutdruckmessgerät im Gebrauchszustand sowohl mechanisch als auch fluidmäßig dauerhaft mit dem proximalen Ende des Ablationskatheters. Es gibt somit auch eine permanent zusammenhängende Flüssigkeitssäule der Spülflüssigkeit in den einzelnen Abschnitten des Schlauches.

In einer alternativen Ausführung ist die Anordnung mit einem Spülflüssigkeitsschlauch mit einer Umschalt-Ventileinrichtung zur Herstellung einer wechselweisen, temporären Fluidverbindung des Ablationskatheters mit dem Blutdruckmessgerät oder der Spülflüssigkeitsquelle, insbesondere der Spülflüssigkeitspumpe versehen.

Durch die Umschalt-Ventileinrichtung, speziell ein Zweiwegeventil, wird die im Katheter selbst ausgebildete Flüssigkeitssäule fluidmäßig entweder mit der Spülflüssigkeitsquelle oder mit dem Blutdruckmessgerät gekoppelt, um entweder einen Fördervorgang der Spülflüssigkeit oder eine Blutdruckmessung auszuführen. Da somit die Spülflüssigkeitspumpe ventilseitig vom Ende des Ablationskatheters getrennt wird, ist eine spezielle Betriebssteuerung der Pumpe nicht erforderlich, um Blutdruckmessungen ausführen zu können.

Es ist gemäß einer weiteren Ausführung durchaus auch vorsehbar, dass die Förder-/Mess-Steuereinheit mit der Spülflüssigkeitspumpe und dem Blutdruckmessgerät sowie dem verzweigten Spülflüssigkeitsschlauch oder der Umschalt-Ventileinrichtung baulich integriert ist.

Zusätzlich kann bei der Ablationskatheteranordnung ein EKG-Messgerät zur Registrierung von abgefühlten Herzaktionspotentialen vorgesehen sein.

In einer weiteren Ausführung ist eine eingangsseitig mit dem Blutdruckmessgerät und/oder dem EKG-Messgerät verbundene Ablationsauswertungseinheit vorgesehen, die bevorzugt mit dem EKG-Messgerät und/oder dem Blutdruckmessgerät und besonders bevorzugt mit beiden Geräten baulich integriert ist. Diese Ausführung führt logisch das Konzept der Erfindung fort, verschiedene Systemkomponenten möglichst zu integrieren bzw. auf separate Teile soweit als möglich zu verzichten.

Vorteile und Zweckmäßigkeiten der Erfindung ergeben sich im Übrigen aus der Beschreibung anhand der Figuren. Von diesen zeigen:
- Fig. 1: Ablationskatheteranordnung aus dem Stand der Technik in schematischer Darstellung,
- Fig. 2: vergleichende Darstellungen von Vorhof-Blutdruckkurven in verschiedenen Herzrhythmuszuständen,
- Fig. 3: eine erste Ausführungsform der Erfindung in schematischer Darstellung,
- Fig. 4: eine zweite Ausführungsform der Erfindung und
- Fig. 5: eine skizzenartige Darstellung einer Ausgestaltung der Mess- und Auswertungs-komponenten der Anordnung.

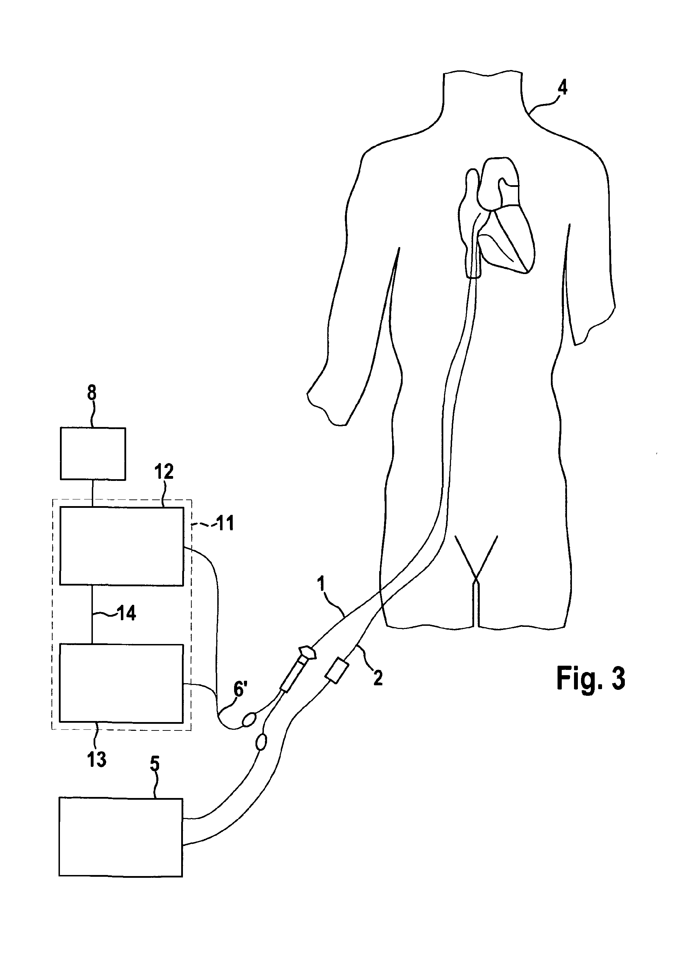

Fig. 3 zeigt eine Ausführungsform der erfindungsgemäßen Lösung, wobei die Darstellung auf Fig. 1 basiert und für gleiche Komponenten auch dieselben Bezugsziffern wie dort verwendet werden. Der Spülflüssigkeitsanschluss des Ablationskatheters 1 ist an ein Gerät bzw. eine Geräteanordnung 11 angeschlossen, welche eine Pumpe 12 und ein Blutdruckmessgerät 13 enthält. Dies können zwei separate Geräte sein, oder es kann ein Gerät mit zwei Modulen sein. Ein modifizierter Spülflüssigkeitsschlauch 6' teilt sich Y-förmig auf und verbindet die Flüssigkeitssäule im Ablationskatheter 1 sowohl mit der Pumpe 12 als auch mit dem Blutdruckmessgerät 13. Auf diese Weise kann gleichzeitig sowohl Flüssigkeit gefördert als auch der Blutdruck gemessen werden.

Um eine zur Förderung simultane Blutdruckmessung zu ermöglichen, teilt die Pumpe 12 dem Blutdruckmessgerät 13 über eine Datenleitung 14 und einen entsprechenden Steuereingang am Messgerät mit, wann und ggf. in welcher Menge und in welcher Art (mit kontinuierlichem, stoßförmigem, wellenförmigem und/oder temperaturabhängigem Druck) die Pumpe 12 gerade Flüssigkeit fördert. Sehr empfindliche Blutdruckmessungen können nur erfolgen, wenn die Pumpe 12 im Moment der Blutdruckmessung die Förderung einstellt. Das wechselweise Pumpen und Blutdruckmessen wird bei einer verbesserten Ausführungsform durch ein entweder in der Pumpe 12 oder im Blutdruckmessgerät 13 eingebautes oder ein separates, in dieser Fig. nicht dargestelltes Steuergerät unter Nutzung der Datenleitung 14 koordiniert.

Vorzugsweise sind die Pumpe 12, das Blutdruckmessgerät 13 und der Y-förmige Spülflüssigkeitsschlauch 6' mit dem Steuergerät und der Datenleitung 14 in einem Gerät integriert.

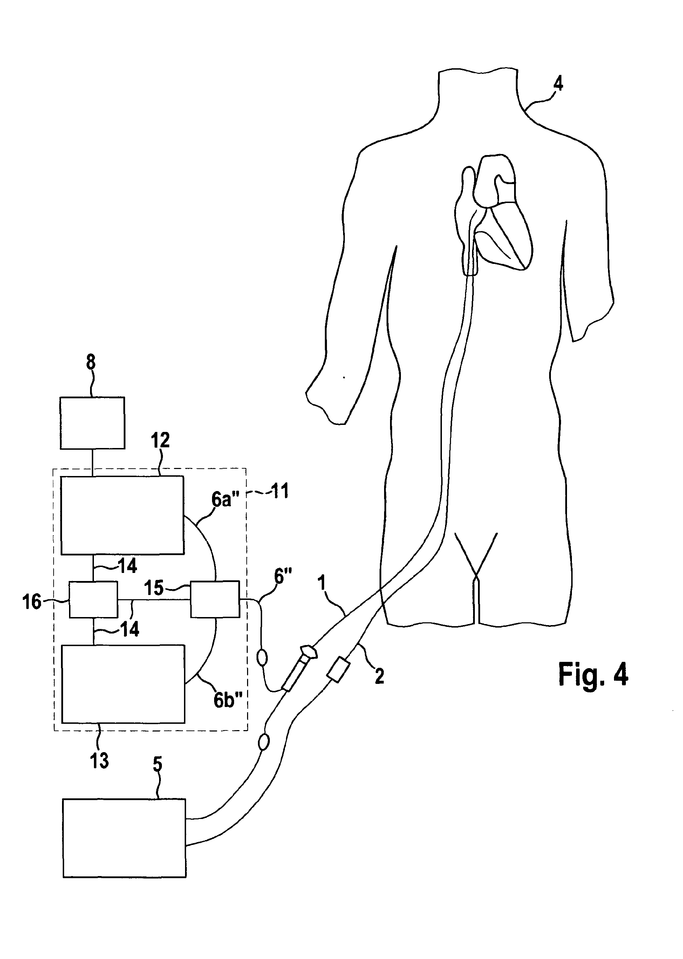

Fig. 4 zeigt eine weitere Ausführungsform der erfindungsgemäßen Lösung. In Ergänzung zu Fig. 3 ist hier anstelle der Y-förmigen Aufteilung am proximalen Ende eines weiter modifizierten Spülschlauches 6" ein Ventil 15 angebracht, welches den Spülflüssigkeitsschlauch über zwei proximale Abschnitte 6a" bzw. 6b" wechselweise mit der Pumpe 12 und mit dem Blutdruckmessgerät 13 verbindet. Ein Steuergerät 16 koordiniert die Ventiltätigkeit mit der Pumpfunktion und dem Blutdruckmessen über diverse Datenleitungen 14. Vorteil dieser Ausführungsform ist die vollständige Entkopplung von Pumpfunktion und Blutdruckmessen, so dass erstere die letztere Funktion nicht stören kann.

Vorzugsweise sind die Pumpe 12, das Blutdruckmessgerät 13, das Ventil 15 und der Y-förmige Spülflüssigkeitsschlauch 6" mit dem Steuergerät 16 und den Datenleitungen 14 in einem Gerät integriert.

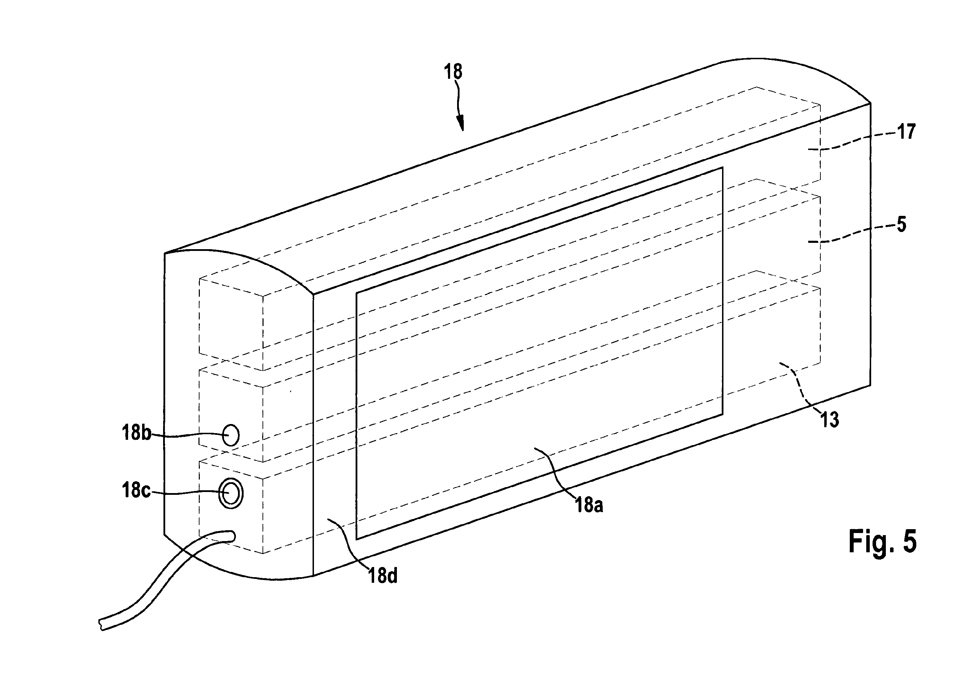

Fig. 5 zeigt eine Ausführung, bei der eine Ablationsauswertungseinheit 17 sowohl mit dem EKG-Messgerät 5 als auch mit dem Blutdruckmessgerät 13 zu einem Mess- und Auswertungsgerät 18 mit einem Anzeigeschirm 18a zusammengefasst ist. Da auch die Eingänge 18b und 18c des EKG-Messgerätes 5 und des Blutdruckmessgerätes 13 eng benachbart zueinander im Gehäuse 18d des Kombinationsgerätes 18 vorgesehen sind, lassen sich auch die Zuleitungen zu beiden Eingängen vom proximalen Ende des Ablationskatheters in einem (nicht gezeigten) Messschlauch 19 zusammenfassen.

## Patentansprüche

1. Ablationskatheteranordnung zur Ablation von Herzgewebe zur Therapie einer Herzrhythmusstörung, insbesondere von Vorhofflattern oder -flimmern, umfassend einen Ablationsgenerator zur Bereitstellung von Energie in zur Ausführung der Ablation geeigneter Form, insbesondere als Hochfrequenzspannung oder in Form eines Kältemittels,
eine Spülflüssigkeitsquelle zur Bereitstellung einer Flüssigkeit zum Spülen des Behandlungsbereiches, in dem die Ablation ausgeführt wird, wobei die Spülflüssigkeitsquelle eine Spülflüssigkeitspumpe aufweist,
eine mit dem Ablationsgenerator und der Spülflüssigkeitsquelle verbindbaren Ablationskatheter mit einem Ablationsbereich, mindestens einer Spülöffnung sowie einem Spülkanal zum Zuführen von Spülflüssigkeit in den Behandlungsbereich,
ein mit dem Spülkanal des Ablationskatheters in Fluidverbindung stehendes und zur Erfassung des intrakardialen Blutdrucks über die Flüssigkeitssäule der Spülflüssigkeit ausgebildetes Blutdruckmessgerät,
**dadurch gekennzeichnet, dass** die Anordnung zusätzlich eine Förder-/Mess-Steuereinheit zur Steuerung einer zeitlich abgestimmten Förderung von Spülflüssigkeit und Messung des Blutdrucks umfasst.

2. Ablationskatheteranordnung nach Anspruch 1, **dadurch gekennzeichnet, dass** die Förder-/Mess-Steuereinheit in die Spülflüssigkeitspumpe integriert ist.

3. Ablationskatheteranordnung nach Anspruch 1, **dadurch gekennzeichnet, dass** die Förder-/Mess-Steuereinheit in das Blutdruckmessgerät integriert ist.

4. Ablationskatheteranordnung nach Anspruch 1, **dadurch gekennzeichnet, dass** die Förder-/Mess-Steuereinheit als separate Einheit ausgeführt und mit der Spülflüssigkeitspumpe und dem Blutdruckmessgerät über Mittel zur Datenkommunikation, insbesondere Signalleitungen, verbindbar ist.

5. Ablationskatheteranordnung nach einem der vorangehenden Ansprüche, **dadurch gekennzeichnet, dass** sie einen verzweigten Spülflüssigkeitsschlauch zur dauerhaften Fluidverbindung des Ablationskatheters sowohl mit dem Blutdruckmessgerät als auch der Spülflüssigkeitsquelle, insbesondere der Spülflüssigkeitspumpe umfasst.

6. Ablationskatheteranordnung nach einem der Ansprüche 1 bis 4, **dadurch gekennzeichnet, dass** sie einen Spülflüssigkeitsschlauch mit einer Umschalt-Ventileinrichtung zur Herstellung einer wechselweisen, temporären Fluidverbindung des Ablationskatheters mit dem Blutdruckmessgerät oder der Spülflüssigkeitsquelle, insbesondere der Spülflüssigkeitspumpe umfasst.

7. Ablationskatheteranordnung nach einem der vorangehenden Ansprüche, wobei die Förder-/Mess-Steuereinheit mit der Spülflüssigkeitspumpe und dem Blutdruckmessgerät sowie dem verzweigten Spülflüssigkeitsschlauch oder der Umschalt-Ventileinrichtung baulich integriert ist.

8. Ablationskatheteranordnung nach einem der vorangehenden Ansprüche, **dadurch gekennzeichnet, dass** sie zusätzlich ein EKG-Messgerät zur Registrierung von abgefühlten Herzaktionspotentialen umfasst.

9. Ablationskatheteranordnung nach einem der vorangehenden Ansprüche, **dadurch gekennzeichnet, dass** sie eine eingangsseitig mit dem Blutdruckmessgerät und/oder dem EKG-Messgerät verbundene Ablationsauswertungseinheit umfasst, die bevorzugt mit dem Blutdruckmessgerät und/oder dem EKG-Messgerät und besonders bevorzugt mit beiden Geräten baulich integriert ist.

## Claims

1. An ablation catheter arrangement for ablation of cardiac tissue for treatment of a cardiac arrhythmia, in particular atrial flutter or atrial fibrillation, comprising an ablation generator for providing energy in a form suitable for performing the ablation process, in particular as high-frequency voltage or in the form of a coolant, a irrigation liquid source for providing a liquid for irrigation the treatment region, in which the ablation is performed, wherein the irrigation liquid source has a irrigation liquid pump,
an ablation catheter that can be connected to the ablation generator and the liquid irrigation source, comprising an ablation region, at least one irrigation opening and a irrigation channel for feeding irrigation liquid into the treatment region,
a blood pressure measurement device in fluid connection with the irrigation channel of the ablation catheter and designed to measure the intracardiac blood pressure via the liquid column of the irrigation liquid,
**characterised in that** the arrangement additionally comprises a supply/measurement control unit for controlling a supply of irrigation liquid and measurement of the blood pressure over time.

2. The ablation catheter arrangement according to Claim 1, **characterised in that** the supply/measurement control unit is integrated into the irrigation liquid pump.

3. The ablation catheter arrangement according to Claim 1, **characterised in that** the supply/measurement control unit is integrated into the blood pressure measurement device.

4. The ablation catheter arrangement according to Claim 1, **characterised in that** the supply/measurement control unit is formed as a separate unit and can be connected to the liquid irrigation pump and to the blood pressure measurement device via means for data communication, in particular signal lines.

5. The ablation catheter arrangement according to one of the preceding claims, **characterised in that** it comprises a branched irrigation liquid tube for permanent fluid connection of the ablation catheter both to the blood pressure measurement device and to the irrigation liquid source, in particular the irrigation liquid pump.

6. The ablation catheter arrangement according to one of Claims 1 to 4, **characterised in that** it comprises a irrigation liquid tube with a switch-over valve device for producing an alternating, temporary fluid connection of the ablation catheter to the blood pressure measurement device or to the irrigation liquid source, in particular the irrigation liquid pump.

7. The ablation catheter arrangement according to one of the preceding claims, wherein the supply/measurement unit is structurally integrated with the irrigation liquid pump and the blood pressure measurement device and also with the branched irrigation liquid tube or the switch-over valve device.

8. The ablation catheter arrangement according to one of the preceding claims, **characterised in that** it additionally comprises an ECG measurement device for recording sensed cardiac action potentials.

9. The ablation catheter arrangement according to one of the preceding claims, **characterised in that** it comprises an ablation evaluation unit, which is connected on the input side to the blood pressure measurement device and/or the ECG measurement device and is preferably structurally integrated with the blood pressure measurement device and/or the ECG measurement device and particularly preferably with both devices.

## Revendications

1. Ensemble de cathéter d'ablation pour l'ablation de tissus cardiaques dans une thérapie des troubles du rythme cardiaque, en particulier le flutter auriculaire ou la fibrillation auriculaire, comprenant un générateur d'ablation pour l'apport d'énergie sous une forme appropriée pour l'exécution de l'ablation, en particulier sous la forme d'une tension haute fréquence ou d'un agent réfrigérant,
une source de liquide d'irrigation pour l'apport d'un liquide destiné à rincer la zone de traitement dans laquelle se déroule l'ablation, la source de liquide d'irrigation comportant une pompe de liquide d'irrigation,
un cathéter d'ablation apte à être relié au générateur d'ablation et à la source de liquide d'irrigation, au moins une ouverture d'irrigation et un canal d'irrigation pour l'alimentation du liquide d'irrigation dans la zone de traitement,
un appareil de mesure de la pression artérielle mis en liaison fluidique avec le canal d'irrigation du cathéter d'ablation et conçu pour détecter la pression artérielle intracardiaque par la colonne de liquide du liquide d'irrigation,
**caractérisé en ce que** l'ensemble comprend en outre une unité de commande de transport/mesure pour la commande d'un transport du liquide d'irrigation et de la mesure de la pression artérielle de façon déterminée dans le temps.

2. Ensemble de cathéter d'ablation selon la revendication 1, **caractérisé en ce que** l'unité de commande de transport/mesure est intégrée dans la pompe de liquide d'irrigation.

3. Ensemble de cathéter d'ablation selon la revendication 1, **caractérisé en ce que** l'unité de commande de transport/mesure est intégrée dans l'appareil de mesure de la pression artérielle.

4. Ensemble de cathéter d'ablation selon la revendication 1, **caractérisé en ce que** l'unité de commande de transport/mesure est conçue comme une unité séparée et peut être reliée à la pompe de liquide d'irrigation et à l'appareil de mesure de la pression artérielle par des moyens pour la communication de données, en particulier des lignes de transmission de signaux.

5. Ensemble de cathéter d'ablation selon l'une des revendications précédentes, **caractérisé en ce qu'**il comprend un tuyau de liquide d'irrigation ramifié, pour la liaison fluidique continue du cathéter d'ablation avec l'appareil de mesure de la pression artérielle ainsi qu'avec la source de liquide d'irrigation, en particulier avec la pompe de liquide d'irrigation.

6. Ensemble de cathéter d'ablation selon l'une des revendications 1 à 4, **caractérisé en ce qu'**il comprend un tuyau de liquide d'irrigation avec un dispositif de soupape de commutation, pour la mise en place d'une liaison fluidique temporaire alternée entre le cathéter d'ablation et l'appareil de mesure de la pression artérielle ou la source de liquide d'irrigation, en particulier la pompe de liquide d'irrigation.

7. Ensemble de cathéter d'ablation selon l'une des revendications précédentes, dans lequel l'unité de commande de transport/mesure est intégrée, quant à sa construction, avec la pompe de liquide d'irrigation et l'appareil de mesure de la pression artérielle, ainsi qu'avec le tuyau de liquide d'irrigation çage ramifié ou le dispositif de soupape de commutation.

8. Ensemble de cathéter d'ablation selon l'une des revendications précédentes, **caractérisé en ce qu'**il comprend en outre un appareil de mesure ECG pour l'enregistrement de potentiels d'activité cardiaque captés.

9. Ensemble de cathéter d'ablation selon l'une des revendications précédentes, **caractérisé en ce qu'**il comprend une unité d'évaluation de l'ablation relié côté entrée à l'appareil de mesure de la pression artérielle et/ou à l'appareil de mesure ECG, laquelle est de préférence intégrée, quant à sa construction, avec l'appareil de mesure de la pression artérielle et/ou avec l'appareil de mesure ECG et de façon encore plus préférée avec les deux appareils.
